# EUROPÄISCHE PATENTSCHRIFT

(11) **EP 2 407 181 B1**
(45) Veröffentlichungstag und Bekanntmachung des Hinweises auf die Patenterteilung: **17.05.2017**
(21) Anmeldenummer: 11172637.8
(22) Anmeldetag: 05.07.2011
(51) Int. Cl.: A61L 2/20, B65B 55/02, B67C 7/00, B01B 1/00

(54) **Verdampfer zum Sterilisieren von Kunststoffbehältnissen**
Evaporator for sterilising plastic containers
Evaporateur pour stérilisation de récipients en matière plastique

(30) Priorität: 13.07.2010 DE 102010027076
(43) Veröffentlichungstag der Anmeldung: 18.01.2012
(73) Patentinhaber: Krones AG, 93073 Neutraubling (DE)
(72) Erfinder: Laumer, Roland, 93047 Regensburg (DE); Knott, Josef, 84069 Walkenstetten/Schierling (DE); Söllner, Jürgen, 93176 Beratzhausen (DE)
(74) Vertreter: Bittner, Bernhard

(56) Entgegenhaltungen:
- EP-A1- 2 286 846
- WO-A1-02/066082
- WO-A1-03/082355
- WO-A1-2009/132686
- WO-A2-2009/013226
- DE-U1-202010 010 223
- GB-A- 2 395 904

## Beschreibung

Die vorliegende Erfindung bezieht sich auf einen Verdampfer zum Sterilisieren von Behältnissen und insbesondere von Kunststoffbehältnissen. Derartige Verdampfer sind aus dem Stand der Technik bekannt. Dabei wird üblicherweise Wasserstoffperoxid (H₂O₂) innerhalb des Verdampfers verdampft und gemeinsam mit einem zugeführten Luftstrom in ein zu sterilisierendes Behältnis geführt. Durch dieses Einführen des Gemisches in das Behältnis kann dessen Innenwandung gereinigt bzw. sterilisiert werden.

Bei derartigen Verdampfern ist es jedoch erforderlich, die Menge des jeweils eingespritzten oder dem Behältnis zuzuführenden H₂O₂ präzise zu regulieren und dafür Sorge zu tragen, dass pro Behältnis jeweils die gleiche Menge an H₂O₂ zugeführt wird.WO 2009/013226 offenbart einen Verdampfer zum Sterilisieren von Behältnissen mit einem Gehäuse mit einer ersten in dem Gehäuse angeordneten Öffnung, um dem Gehäuse ein gasförmiges Medium zuzuführen, mit einer Einspritzeinrichtung, um in das Gehäuse eine Substanz einzuspritzen, welche Wasserstoffperoxid enthält, mit einer innerhalb des Gehäuses angeordneten Heizeinrichtung zum Verdampfen des Wasserstoffperoxids und mit einer zweiten in dem Gehäuse angeordneten Öffnung, um ein Gemisch aus dem gasförmigen Medium und dem verdampften Wasserstoffperoxid aus dem Gehäuse abzuführen, wobei die Heizeinrichtung eine von einem fliessfähigen Medium durchströmbare Leitung aufweist, wobei diese Leitung zum Erwärmen der Heizeinrichtung dient. Aus der WO 2007/003313 sind ein Verfahren und eine Vorrichtung zum Überwachen eines Verdampfers bekannt. Bei diesem Verfahren wird auf einen erwärmten Heizkörper eine Flüssigkeit aufgebracht und verdampft und weiterhin ist seitlich an diesen Verdampfer eine Rohrleitung vorgesehen, durch welche hindurch erwärmte und sterile Luft von der Seite her in den Verdampfer bzw. dessen Gehäuse eingeleitet wird. Damit wird die Luftströmung hier senkrecht zu der Einspritzrichtung des Wasserstoffperoxids geführt. Diese Vorrichtung erlaubt eine effektive Zuführung von verdampften Wasserstoffperoxid in das Behältnis. Durch die Art der Zuführung ist jedoch eine exakte Regulierung der genauen Menge an Wasserstoffperoxid nur schwer möglich.

Die DE 100 40 861 A1 beschreibt eine Vorrichtung zum Sterilisieren von Packungen mit Wasserstoffperoxid. Dabei wird das Wasserstoffperoxid in Längsrichtung an einer warmen Oberfläche vorbeigeführt. Auf diese Weise wird das mit dem Wasserstoffperoxid in nebelform versetzte Trägergas schon an dieser warmen Oberfläche verdampft. Auch bei dieser Vorrichtung ist eine genaue Regulierung der zugeführten Menge an Wasserstoffperoxid nur schwer möglich.

Aus der EP 0 991 434 B1 ist ein multiples Flammpunkt-Verdampfungssystem bekannt. Auch wird in einen Verdampfer sowohl Wasserstoffperoxid als auch ein Trägergas eingeführt, wobei das Trägergas hier ebenfalls quer bezüglich der Einspritzrichtung des Wasserstoffperoxids fließt.

Die US 5,879,648 beschreibt eine Vorrichtung zum Desinfizieren von Behältern. Dabei wird in einen Verdampfer sowohl Wasserstoffperoxid als auch über parallel verlaufende Öffnungen ein Gas eingeführt. Auch bei dieser Vorrichtung ist eine Feinregulierung des aus dem Verdampfer in die Behältnisse gelangenden Gemisches nur schwer möglich.

Aus der DE 10 2007 034 205 ist ebenfalls ein Verdampfer bekannt, bei dem sowohl ein Sterilisationsmittel mittels einer elektrischen Heizeinrichtung verdampft wird. Derartige Heizeinrichtungen sind jedoch in der Herstellung und insbesondere auch im Betrieb relativ teuer.

Der vorliegenden Erfindung liegt daher die Aufgabe zugrunde, einen Verdampfer zu schaffen, der insbesondere im Betrieb kostengünstig ist. Daneben soll ein Verdampfer zu Verfügung gestellt werden. der eine sehr genaue Regulierung des aus diesem austretenden Wasserstoffperoxid-Gasgemisches erlaubt.

Dies wird erfindungsgemäß durch einen Verdampfer nach Anspruch 1 sowie ein Verfahren zum Sterilisieren von Behältnissen nach Anspruch 16 erreicht. Vorteilhafte Ausführungsformen und Weiterbildungen sind Gegenstand der Unteransprüche.

Der erfindungsgemäße Verdampfer zum Sterilisieren von Behältnissen ist in Anspruch 1 definiert. Erfindungsgemäß weist die Heizeinrichtung eine von einem fließfähigen Medium durchströmbare Leitung auf, wobei diese Leitung zum Erwärmen der Heizeinrichtung dient und von der Substanz - insbesondere unmittelbar - beaufschlagbar ist.

Es werden daher Weiterentwicklungen, insbesondere hinsichtlich der Heiz- oder Erwärmungseinrichtung vorgeschlagen. Im Stand der Technik werden für die Heizeinrichtung üblicherweise hochverdichtete Heizpatronen verwendet. Im Rahmen der vorliegenden Erfindung wird vorgeschlagen, die Erwärmung mittels eines fließfähigen Mediums, insbesondere Dampf, zu erreichen. Dabei wird das zugeführte fließfähige Medium eine Verdampfungseinrichtung erhitzen. Auf diese Verdampfungs- bzw. Heizeinrichtung wird dann das flüssige Sterilisationsmedium, insbesondere H₂O₂ aufgebracht und dabei verdampft. Der entstehende H₂O₂ Dampf wird dann durch die ebenfalls eingeleitete Luft aufgenommen und aus dem Verdampfer abtransportiert. Bei dem fließfähigen Medium handelt es sich insbesondere um Dampf, es könnte jedoch auch ein anderes Medium, wie etwa Öl vorgesehen sein.

Die Vorteile eines derart mit Fließmittel betätigten Verdampfers liegen vor allem in der günstigeren Energie sowie der wesentlich einfacheren Regelung dieses Verdampfers und ferner auch darin, dass der Verdampfer nicht überhitzen kann. Unter einer - insbesondere unmittelbar - beaufschlagbaren Leitung wird verstanden, dass das Medium direkt an die Leitung, insbesondere eine Außenoberfläche der Leitung gelangen kann. Damit erwärmt die Leitung nicht eine Heizfläche, an der die Substanz verdampfen kann, sondern die Leitung, insbesondere deren Außenoberfläche bildet insbesondere selbst eine Heizfläche aus.

Bei einer weiteren vorteilhaften Ausführungsform weist die Einspritzeinrichtung eine Düse auf, welche das fließfähige Medium auf eine Außenoberfläche der Leitung aufbringt. Diese Düse kann dabei das Sterilisationsmedium auch besonders vorteilhaft zerstäuben, um auf diese Weise den Verdampfungsprozess effizienter zu gestalten. Bei einer weiteren vorteilhaften Ausführungsform ist die Leitung wenigstens abschnittsweise spiralförmig ausgebildet. Durch diese spiralförmige Ausbildung kann die Oberfläche, auf welche das Sterilisationsmedium aufgebracht werden kann, vergrößert und auf diese Weise die Effizienz gesteigert werden.

Bei einer weiteren vorteilhaften Ausführungsform bildet die Leitung wenigstens abschnittsweise eine kegelförmige bzw. kegelstumpfförmige Oberfläche auf. Dabei könnten beispielsweise die Rohrleitungen spiralförmig gewunden werden und beispielsweise von oben nach unten einen sich verringerten Querschnitt aufweisen. Durch diese kegelförmige Oberfläche ist es weiter möglich, die Oberfläche der gesamten Heizeinrichtung zu vergrößern. Besonders vorteilhaft ist eine spiralförmig sich nach unten verringernde Anordnung der besagten Leitung bzw. eine sich verringernde Wendel vorgesehen. Vorzugsweise liegen die einzelnen Wendel der Leitung aneinander, sodass die Substanz keine Zwischenräume dieser Wendel passieren kann. Damit ist vorteilhaft hier die Leitung als gerolltes oder gewickeltes Rohr ausgebildet. Es wäre jedoch auch möglich, dass die Leitung dadurch ausgebildet ist, dass an einer Außenoberfläche eines Grundkörpers ein spiralförmiger Steg angeordnet ist und eine zweite Wand auf diesem Steg aufliegt, sodass sich insbesondere ein spiralförmiger Kanal ergibt.

Bei einer weiteren vorteilhaften Ausführungsform ist ein weiterer Bestandteil des Verdampfers erwärmbar. So könnte beispielsweise die Vorrichtung unterhalb der Leitung einen beheizbaren Abschnitt aufweisen. Dieser beheizbare Abschnitt könnte dabei elektrisch geheizt werden, beispielsweise mit Heizpatronen, aber auch durch die Verwendung eines fließfähigen Mediums.

Bei einer weiteren vorteilhaften Ausführungsform ist wenigstens ein weiterer Bestandteil des Gehäuses auch durch ein fließfähiges Medium erwärmbar. So könnte beispielsweise die Vorrichtung einen doppelwandigen Boden aufweisen, und ein fließfähiges erwärmendes Medium könnte im Zwischenraum zwischen diesen beiden Böden eingeführt werden. So wäre es beispielsweise möglich, dass der Verdampfer mit einem doppelwandigen Klöpperboden ausgeführt ist und zusätzlich die oben beschriebene konische Rohrwendel im Inneren des Verdampfers vorgesehen ist. Eine Versorgung mit dem fließfähigen Medium, insbesondere eine Dampfversorgung wird dabei vorteilhaft mit einer gemeinsamen Zuleitung bewerkstelligt. Diese Zuleitung kann sich dann in zwei Flansche aufzweigen, wobei es möglich ist, dass das Kondensat der Wendel direkt in den doppelwandigen Klöpperboden geleitet und dort durch einen Kondensatabscheider ausgeleitet wird.

Der Hauptteil der zur Verdampfung des H₂O₂ benötigten Energie wird dabei vorteilhaft durch die Leitung zugeführt. Der Vorteil dieser Leitung liegt einerseits in der großen Übertragungsfläche, welche dieses gewickelte Rohr bietet und daneben auch in der wesentlich geringeren Wandstärke gegenüber dem Boden bzw. Klöpperboden. Auf diese Weise kann im Verhältnis zu dem Klöpperboden wesentlich mehr Energie übertragen werden. Hierbei wäre es auch möglich, eine Ausführungsform zu schaffen, welche ohne die besagte innere Wendel auskommt, diese wäre jedoch in ihren Abmessungen erheblich größer.

In einer weiteren Ausführungsform wäre es auch möglich, die Begleitheizung deutlich zu reduzieren und diese Begleitheizung mit einer anderen Energieform vorzugsweise elektrischer Energie, zu versorgen. Da der Hauptteil der benötigten Energie über die Dampfheizung zugeführt wird, bleiben auch hier die Verluste gering. Auf diese Weise wäre eine Bauform denkbar, die ohne Druckbehälter auskommt, was wiederum die Herstellungskosten verringern könnte.

Bei einer weiteren vorteilhaften Ausführungsform ist die Einspritzeinrichtung als Ringdüse ausgebildet. So könnten beispielsweise eine oder auch mehrere Düsen vorgesehen sein, wobei vorteilhaft über die besagte Ringdüse das H₂O₂ direkt auf den dampfführenden Leitungsteil aufgebracht und dort verdampft wird. Wie oben erwähnt, kann dabei zusätzlich auch das Gehäuse oder ein wesentlicher Teil des Gehäuses des Verdampfers so ausgeführt werden, dass es erwärmt werden kann. Der Vorteil dieses erwärmten Gehäuses bzw. Gehäuseteils liegt darin, dass auch von den Leitungen abtropfendes H₂O₂ noch verdampft wird.

Vorteilhaft ist unterhalb der Leitung ein doppelwandiger Boden vorgesehen, wobei zwischen den Wänden dieses Bodens ein fließfähiges Medium führbar ist. Auf diese Weise kann, wie oben erwähnt, auch im Bereich des Bodens des Verdampfers noch ein zusätzliches Verdampfen des Sterilisationsmediums durchgeführt werden. Vorteilhaft schließt sich dabei der doppelwandige Boden in einer Strömungsrichtung des fließfähigen Mediums an die Leitung an. Auf diese Weise kann das fließfähige Medium bzw. der Dampf, der bereits die Leitung erwärmt hat im Nachgang auch noch den besagtem Boden erwärmen.

Vorteilhaft erstreckt sich eine Längsrichtung der Einspritzeinrichtung in einer vertikalen Richtung und an die erste Öffnung schließt sich ein Rohrabschnitt an, der sich wenigstens abschnittsweise in der Längsrichtung der Einspritzeinrichtung erstreckt, wobei die Einspritzeinrichtung wenigstens abschnittsweise innerhalb dieses Rohrabschnitts verläuft.

Bei der Einspritzeinrichtung handelt es sich bevorzugt um eine lanzenförmige Einspritzeinrichtung und die Längsrichtung der Einspritzeinrichtung ist durch die Längsrichtung dieser Lanze definiert. Bevorzugt handelt es sich bei der eingespritzten Substanz um Wasserstoffperoxid. Dieses kann dabei sowohl in flüssiger Form als auch in nebelartiger Konsistenz eingespritzt werden. Es wären jedoch auch andere als lanzenförmige Einspritzeinrichtungen denkbar. Bei anders gestalteten Einspritzeinrichtungen ist deren Längsrichtung auch durch die Strömungsrichtung des H₂O₂ beim Austritt durch eine Düse der Einspritzeinrichtung definiert.

Durch die Anordnung der Einspritzeinrichtung innerhalb des Rohrabschnitts kann weiterhin erreicht werden, dass das aus dem Rohrabschnitt austretende Gas im Wesentlichen gleichförmig die Einspritzeinrichtung umgibt. Auf diese Weise kann ebenfalls eine gleichmäßige Anreicherung des Gases mit dem Wasserstoffperoxid bewirkt werden. Bei einer vorteilhaften Ausführungsform erstreckt sich der Rohrabschnitt innerhalb des Gehäuses wenigstens abschnittsweise in der Längsrichtung der Einspritzeinrichtung. Dies bedeutet, dass sich der Rohrabschnitt ausgehend von der ersten Öffnung nach innen und in der Längsrichtung der Einspritzeinrichtung erstreckt.

Weiterhin weist bevorzugt der Rohrabschnitt eine erste Austrittsöffnung auf, die derart angeordnet ist, dass das gasförmige Medium in einer zu der Längsrichtung parallelen Richtung aus dem Rohrabschnitt austritt. Dies bedeutet, dass es sich bei demjenigen Rohrabschnitt, der parallel zu der Längsrichtung der Einspritzrichtung ist, um einen Endabschnitt dieses Rohrabschnitts handelt.

Bei einer weiteren vorteilhaften Ausführungsform weist der Rohrabschnitt eine zweite Austrittsöffnung auf, die zwischen der in dem Gehäuse angeordneten Öffnung (des Gehäuses) und der ersten Austrittsöffnung vorgesehen ist. Auf diese Weise kann ein Teilstrom auch in einer anderen Richtung als der Längsrichtung der Einspritzeinrichtung ausgeführt werden.

Vorzugsweise ist diese zweite Austrittsöffnung derart angeordnet, dass das durch diese Austrittsöffnung austretende gasförmige Medium unmittelbar in Richtung der zweiten Öffnung (des Gehäuses) strömt. Unter unmittelbar wird dabei verstanden, dass die Strömung ohne umgelenkt zu werden, in Richtung der zweiten Öffnung strömt. Vorzugsweise weist das Gehäuse abgesehen von der ersten Öffnung und der zweiten Öffnung keine Öffnung auf, durch welche hindurch ein gasförmiges Medium in das Gehäuse hinein oder aus dem Gehäuse heraustreten kann.

Vorzugsweise sind die erste Öffnung in dem Gehäuse und die zweite Öffnung in dem Gehäuse auf gegenüberliegenden Seiten beispielsweise auf gegenüberliegenden Seiten in der Ummantelung des Gehäuses oder auch auf gegenüberliegenden Seiten in Längsrichtung der Einspritzeinrichtung angeordnet.

Vorteilhaft ist die erste Öffnung in einer Umfangswand des Gehäuses angeordnet. Bei einer weiteren vorteilhaften Ausführungsform weist der Rohrabschnitt einen gekrümmten Bereich auf, wobei dieser gekrümmte Bereich in einer Ausführungsform im Inneren des Gehäuses verläuft.

Bevorzugt ist bei dieser Ausführungsform die zweite Austrittsöffnung des Rohrabschnitts in dem gekrümmten Bereich desselben angeordnet.

Bei einer weiteren vorteilhaften Ausführungsform weist die Einspritzeinrichtung eine Austrittsdüse für das Wasserstoffperoxid auf und diese Austrittsdüse ist unterhalb der ersten Austrittsöffnung des Rohrabschnitts angeordnet. Dies bedeutet, dass der Rohrabschnitt und die Austrittsdüse nicht auf gleiche Höhe abschließen sondern die Austrittsdüse näher an der Heizeinrichtung ist als die Austrittsöffnung des Rohrabschnitts. Bei einer weiteren Ausführungsform sind an dem Rohrabschnitt Luftführungseinrichtungen vorgesehen, welche einen Teil der Luftströmung an eine Innenwandung des Verdampfers richten. Bevorzugt weist der Rohrabschnitt ein Innenrohr auf, welches die Einspritzeinrichtung konzentrisch umgibt und in wenigstens einem Bereich dieses Innenrohrs ist ein Außenrohr vorgesehen, wobei zwischen diesem Außenrohr und dem Innenrohr die Luftführungseinrichtungen vorgesehen sind. In diesem Falle handelt es sich bei dem Innenrohr um den erfindungsgemäßen Rohrabschnitt. Bei einer weiteren vorteilhaften Ausführungsform sind die Einspritzeinrichtung und der Rohrabschnitt wenigstens abschnittsweise im Wesentlichen konzentrisch zueinander. Weiterhin ist besonders bevorzugt der Verdampfer bzw. dessen Gehäuse im Wesentlichen rotationssymmetrisch bezüglich einer Zentralachse ausgebildet.

Bei einer weiteren vorteilhaften Ausführungsform ist die Einspritzeinrichtung drehbar gegenüber dem Gehäuse angeordnet. Genauer gesagt ist die Einspritzeinrichtung und bevorzugt auch der Rohrabschnitt stationär und das Gehäuse dreht sich diesem gegenüber. Es wäre jedoch auch möglich, die Einspritzeinrichtung fest an dem Gehäuse und dieses stationär anzuordnen und gegenüber dem Gehäuse einen Auslassstutzen zum Abführen des Gemisches drehbar anzuordnen.

Die vorliegende Erfindung ist weiterhin auf eine Anordnung zum Sterilisieren von Behältnissen und insbesondere von Kunststoffbehältnissen gerichtet, die wenigstens einen Verdampfer der oben beschriebenen Art aufweist. Bevorzugt weist diese Anordnung eine Erwärmungseinrichtung zum Erwärmen des gasförmigen Mediums auf. Genauer gesagt wird das gasförmige Medium erwärmt, bevor es in den Verdampfer eintritt.

Die vorliegende Erfindung ist weiterhin auf ein Verfahren zum Sterilisieren von Behältnissen nach Anspruch 10 gerichtet. Es wird daher auch verfahrensseitig vorgeschlagen, die Erwärmung nicht oder nicht nur über eine elektrische Heizeinrichtung durchzuführen, sondern insbesondere auch ein fließfähiges Medium, wie beispielsweise Dampf zu verwenden.

Die vorliegende Erfindung eignet sich insbesondere beim Betrieb von Anlagen zur Sterilisation von Behältnissen, die sehr hohen hygienischen Anforderungen genügen müssen. Insbesondere können durch die Verwendung der Erfindung die Anforderungen der US Behörde FDA (Food and Drug Administration) leichter erfüllt werden.

Weitere Vorteile und Ausführungsformen ergeben sich aus den beigefügten Zeichnungen:

Darin zeigen:
- Fig. 1: eine perspektivische Ansicht eines Verdampfers nach dem Stand der Technik;
- Fig. 2: eine Querschnittsdarstellung des Verdampfers aus Fig. 1;
- Fig. 3: eine Querschnittsdarstellung eines erfindungsgemäßen Verdampfer;
- Fig. 4: einen Verdampfer in einer weiteren Ausführungsform; und
- Fig. 5: einen Verdampfer in einer weiteren Ausführungsform.

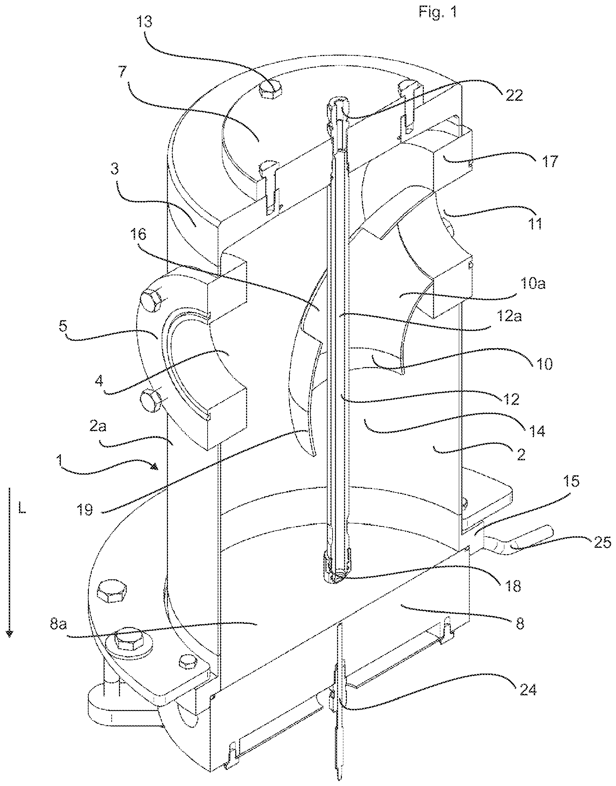

Fig. 1 zeigt einen Verdampfer 1. Dieser Verdampfer weist ein Gehäuse 2 mit einer umlaufenden Wandung 2a und einem Deckelabschnitt 3 auf, der durch einen mit Schrauben 13 aufgeschraubten Deckel 7 verschlossen ist. Nach unten hin ist das Gehäuse durch eine Heizeinrichtung 8 bzw. eine Heizfläche 8a abgeschlossen. Die Ebene dieser Heizfläche steht senkrecht zur Figurenebene. Das Bezugszeichen 12 bezieht sich auf eine Einspritzeinrichtung, deren Längsrichtung sich senkrecht zu der Ebene der Heizfläche 8a erstreckt. Durch diese Einspritzeinrichtung 12, genauer gesagt einen Kanal 12a im Innenraum der Einspritzeinrichtung 12 wird Wasserstoffperoxid in das Gehäuse 2 und in Richtung der Heizeinrichtung 8 geführt. Bei der in Fig. 1 gezeigten Ausführungsform ist die Einspritzeinrichtung 12 konzentrisch mit der Umfangswand 2a angeordnet. Am unteren Ende weist die Einspritzeinrichtung 12 eine Düse 18 auf, über die Wasserstoffperoxid austreten kann und auf die Heizfläche 8a gespritzt wird.

Das Bezugszeichen 24 bezieht sich auf einen Temperatursensor zum Ermitteln der Temperatur der Heizeinrichtung 8. Oberhalb der Heizeinrichtung 8 ist ein Trägerring 15 vorgesehen, auf dem wiederum das Gehäuse 2 abgestützt ist. Bevorzugt ist dieser Trägerring 15 aus einem Material hergestellt, welches einen geringen Wärmeleistungsquotienten aufweist, damit sich über die von der Heizeinrichtung 8 abgegebene Wärme das Gehäuse 2 nicht oder nicht wesentlich erwärmt.

Das Bezugszeichen 11 bezieht sich auf eine erste Öffnung (Eintrittsöffnung) in dem Gehäuse 2, durch welche hindurch das gasförmige Medium in das Innere des Gehäuses 2 gelangen kann. Die erste Öffnung 11 ist dabei in einem Flansch 17 angeordnet, der an der Gehäusewandung 2a befestigt ist. Im Inneren des Gehäuses 2 schließt sich an die erste Öffnung 11 ein Rohrabschnitt 10 an. Dieser Rohrabschnitt 10 weist einen gekrümmten Bereich 10a und eine erste Austrittsöffnung 14, durch welche hindurch das gasförmige Medium austreten kann, auf. Damit erfolgt die Strömungsführung der erwärmten Luft abschnittsweise parallel zu der Wasserstoffperoxidzufuhr über die Einspritzeinrichtung 12. Zu diesem Zweck wird die durch die erste Öffnung 11 einströmende Luft in den gekrümmten Abschnitt 10a umgelenkt.

Das Bezugszeichen 16 bezieht sich auf eine zweite Austrittsöffnung in dem Rohrabschnitt 10. Durch diese Öffnung tritt einerseits die Einspritzeinrichtung 12 hindurch. Auf diese Weise kann die Einspritzeinrichtung wenigstens teilweise innerhalb des Rohrabschnitts 10 geführt werden. Daneben kann durch die zweite Austrittsöffnung 6 auch ein Anteil des gasförmigen Mediums aus dem Rohrabschnitt austreten und in Richtung einer Austrittsöffnung 4 gelangen. Über die Austrittsöffnung 4, die ebenfalls mittels eines Flansches 5 an der Gehäusewandung 2a angeordnet ist, kann das in dem Verdampfer entstehende Gasgemisch abgeführt und anschließend den zu sterilisierenden Behältnissen zugeführt werden.

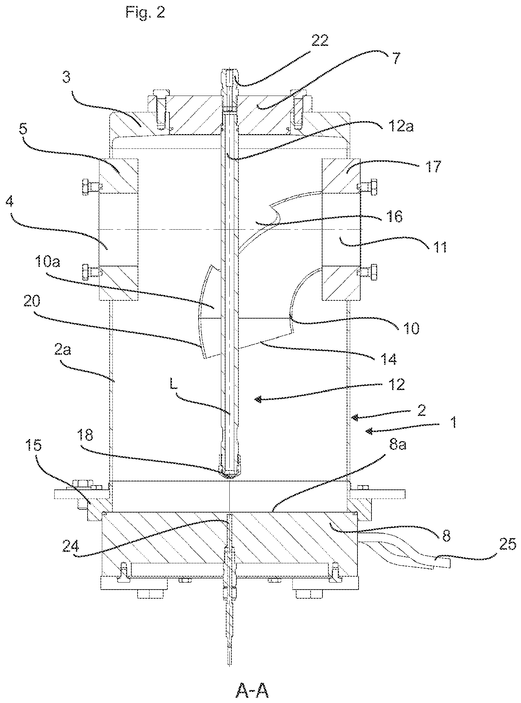

Fig. 2 zeigt eine Querschnittsdarstellung eines Verdampfers aus Figur 1. Man erkennt, dass die Düse 18 der Einspritzeinrichtung 12 wesentlich weiter in das Innere des Gehäuses hineinragt bzw. wesentlich näher an der Heizeinrichtung 8 angeordnet ist als die erste Austrittsöffnung 14 des Rohrabschnitts 10. In dem Endabschnitt 19 des Rohrabschnitts 10 wird das hindurch tretende gasförmige Medium nicht genau in der Längsrichtung geführt sondern in einem gewissen Winkel hierzu geneigt. Auf diese Weise wird erreicht, dass durch entsprechende Reflexionen des gasförmigen Mediums an der Heizeinrichtung eine besonders vorteilhafte Durchmischung mit dem verdampfenden Wasserstoffperoxid auftritt. Genauer gesagt wird das durch den Rohrabschnitt 10 hindurch tretende gasförmige Medium wieder geringfügig zurück in Richtung der ersten Öffnung 4 geführt. Das Bezugszeichen 22 bezieht sich auf einen Anschluss für eine Wasserstoffperoxidzuleitung. Das Bezugszeichen 25 bezieht sich auf eine Zuleitung für ein Kühlmedium für die Heizeinrichtung 8.

Man erkennt, dass die zweite Austrittsöffnung 16 in dem Rohrabschnitt 10 derart ausgelegt ist, dass sich ein größerer Abschnitt dieser zweiten Austrittsöffnung 16 rechts bezüglich der Einspritzeinrichtung 12 das heißt der Einspritzlanze 12 befindet und nur ein kleiner Abschnitt links hiervon. Auf diese Weise kann der Rohrabschnitt innerhalb des Gehäuses und damit auch seine Oberfläche gering gehalten werden.

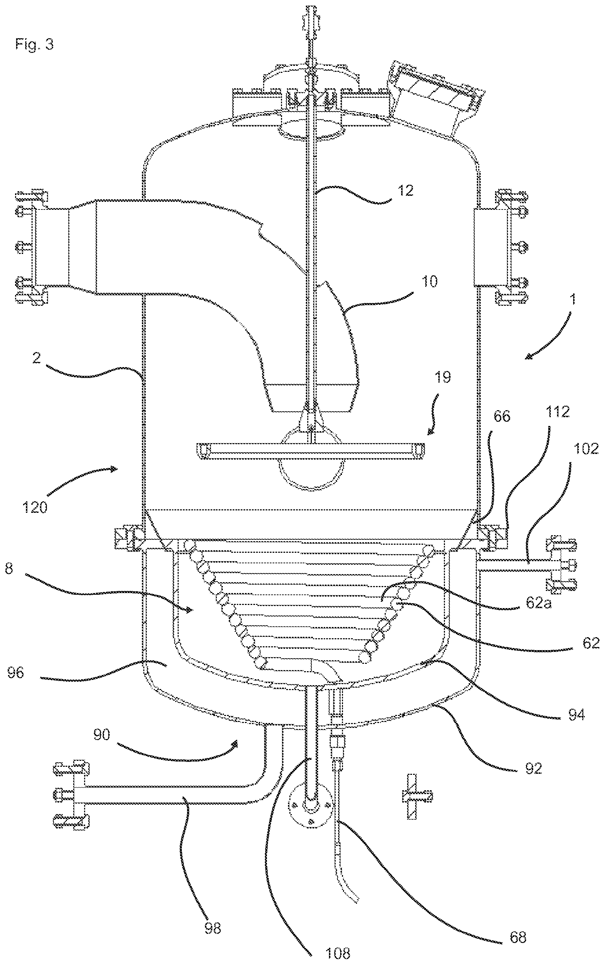

Fig. 3 zeigt eine Darstellung eines erfindungsgemäßen Verdampfers 1. Dieser Verdampfer 1 weist dabei wiederum ein Gehäuse 2 auf, sowie den Rohrabschnitt 10, über den das gasförmige Medium, wie beispielsweise erwärmte Luft, in den Verdampfer eingeführt wird. Die Einspritzeinrichtung 12 mündet hier in eine Ringdüse 19, welche das Wasserstoffperoxid mit einer Vielzahl von in Umfangsrichtung verlaufenden Düsen einspritzt.

Die Heizeinrichtung 8 weist hier eine Leitung 62 auf, welche als Rohrwendel ausgeführt ist, und sich nach oben hin, d. h. in Richtung der Einspritzeinrichtung erweitert. Die Ringdüsen 19 können damit das Sterilisationsmedium auf die Außenoberfläche 62a der Leitung 62, d.h. die Innenoberfläche des aus der Rohrwendel gebildeten Kegelstumpfes aufbringen und dort kann das Medium verdampfen. Alternativ ist auch eine sich nach oben hin verjüngende kegel- oder kegelstumpfförmige Anordnung der Rohrwendel möglich. Auch in dieser Ausführungsform wird erreicht, dass eine möglichst große Heizoberfläche vorliegt. Weiterhin ist eine zylindrische Anordnung der Rohrwendel möglich, wobei dann die Ringdüse 19 vorteilhaft innerhalb des gebildeten Zylinders angeordnet ist oder gar eine zweite Ringdüse zur gleichzeitigen Beaufschlagung einer Außenoberfläche des Zylinders eingesetzt wird.

Über eine nicht gezeigte Zuleitung wird das fließfähige Medium beispielsweise dann der Leitung 62 zugeführt und gelangt anschließend in Fig. 3 von unten nach oben. Am Ende der Leitung 62 kann das fließfähige Medium in einem Zwischenbereich 96 zwischen dem unteren Boden 92 und einem höheren Boden 94 gelangen. Auf diese Weise kann auch insbesondere der Bodenabschnitt 94 von innen erwärmt werden.

Das Bezugszeichen 98 bezieht sich auf die Abführleitung zum Abführen von Kondensat aus dem Boden 90 des Verdampfers 1. Das Bezugszeichen 108 kennzeichnet eine weitere Abführleitung um nicht überschüssiges Desinfektionsmittel aus dem Bereich zwischen der Leitung 62 und dem oberen Boden 94 abzuführen. Dadurch wird erreicht, dass sich in der Heizeinrichtung 8 kein Desinfektionsmittel anstaut und die Bodenheizfläche vollständig als Verdampfungsfläche zur Verfügung steht.

Man erkennt, dass der untere Teil des Verdampfers, d. h. einschließlich der Leitung 62 und der beiden Böden 92 und 94 über eine Verbindungseinrichtung, wie einen Flansch 112 an dem oberen Teil 120 des Gehäuses angeordnet ist. Das Bezugszeichen 66 kennzeichnet Leitbleche welche die Substanz zu der Heizeinrichtung 8 leiten. Das Bezugszeichen 102 kennzeichnet eine Zuführleitung zum Zuführen des Heizmediums in den Zwischenbereich 96 des Bodens 90. Das Bezugszeichen 68 kennzeichnet einen Temperatursensor, der die Oberflächentemperatur des oberen Bodens 94 und somit einer der Heizflächen aufnimmt.

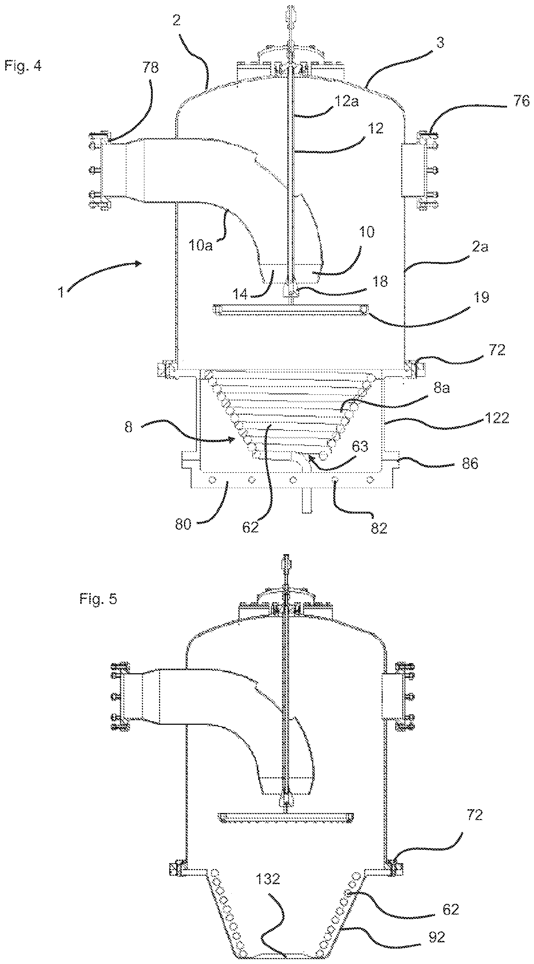

Fig. 4 zeigt eine weitere Ausführungsform eines erfindungsgemäßen Verdampfers. Bei dieser Ausführungsform ist unterhalb der Leitung 62 keine Weiterführung bzw. kein Doppelboden vorgesehen sondern eine elektrische Heizung. Zu diesem Zweck ist eine Vielzahl von Heizpatronen 82 in einer beheizbaren Bodenplatte 80 angeordnet und erwärmt diese. Es wäre auch möglich, dass das Sterilisationsmedium durch eine Öffnung 63, welche durch die Ausgestaltung der kegelförmigen Wendel 62 ausgebildet wird, auf diese elektrische Heizplatte 80 fällt. Die elektrische Heizplatte 80 ist dabei wiederum über einen Flansch von dem Bodenteil 122, in dem auch die Leitung 62 angeordnet ist, trennbar.

Fig. 5 zeigt eine weitere Ausführungsform der erfindungsgemäßen Vorrichtung. Bei dieser Ausführungsform ist keine weitere Zusatzheizung bzw. Begleitheizung vorgesehen. Bei dieser Ausführungsform wird der dampfführende Leitungsteil 62 so ausgeführt, dass vom Leitungsteil 62 abtropfendes H₂O₂ zwangsweise wieder zu dem dampfführenden Leitungsteil 62 zurückgeführt wird. So kann beispielsweise eine Erhebung 132 vorgesehen sein, welche das H₂O₂ wieder in Richtung der Leitung 62 transportiert. Diese Erhebung 132 kann dabei durch entsprechende Gestaltung der Gehäusewand oder auch durch ein Leitblech ausgeführt sein. Daneben wäre hier jedoch auch eine Bauform denkbar, die nicht als Druckbehälter ausgeführt ist.

### Bezugszeichenliste

- 1: Verdampfer
- 2: Gehäuse
- 2a: umlaufende Wandung
- 3: Deckelabschnitt des Gehäuses
- 4: zweite (Austritts-) Öffnung in dem Gehäuse 2
- 5: Flansch
- 7: aufgeschraubter Deckel
- 8: Heizeinrichtung
- 8a: Heizfläche
- 10: Rohrabschnitt
- 10a: gekrümmter Bereich
- 11: erste (Eintritts-) Öffnung in dem Gehäuse 2
- 12: Einspritzeinrichtung
- 12a: Kanal der Einspritzeinrichtung
- 13: Schraube
- 14: erste Austrittsöffnung in dem Rohrabschnitt 10
- 15: Trägerring
- 16: zweite Austrittsöffnung in dem Rohrabschnitt 10
- 17: Flansch
- 18: Düse
- 19: Ringdüse
- 22: Anschluss
- 24: Temperatursensor
- 25: Zuleitung
- 62: Leitung
- 62a: Außenoberfläche
- 63: Öffnung
- 66: Leitblech
- 68: Temperatursensor
- 80: beheizbare Bodenplatte
- 82: Heizpatrone
- 90: Boden
- 92: unterer Boden
- 94: oberer Boden
- 96: Zwischenbereich
- 98: Abführleitung
- 102: Zuführleitung
- 108: weitere Abführleitung
- 112: Flansch
- 120: oberer Teil des Gehäuses
- 122: Bodenteil
- 132: Erhebung

- L: Längsrichtung

## Patentansprüche

1. Verdampfer (1) zum Sterilisieren von Behältnissen mit einem Gehäuse (2), mit einer ersten in dem Gehäuse (2) angeordneten Öffnung (11), um dem Gehäuse (2) ein gasförmiges Medium zuzuführen, mit einer Einspritzeinrichtung (12), um in das Gehäuse (2) eine Substanz einzuspritzen, welche Wasserstoffperoxid (H₂O₂) enthält, mit einer innerhalb des Gehäuses (2) angeordneten Heizeinrichtung (8) zum Verdampfen des Wasserstoffperoxids und mit einer zweiten in dem Gehäuse (2) angeordneten Öffnung (4), um ein Gemisch aus dem gasförmigen Medium und dem verdampften Wasserstoffperoxid aus dem Gehäuse (2) abzuführen,
**dadurch gekennzeichnet, dass**
die Heizeinrichtung (8) eine von einem fließfähigen Medium durchströmbare Rohrleitung (62) aufweist, wobei diese Leitung (62) zum Erwärmen der Heizeinrichtung (8) dient und von der Substanz unmittelbar beaufschlagbar ist derart, dass die Wasserstoffperoxid (H₂O₂) enthaltende Substanz direkt an eine Außenoberfläche der Leitung gelangen kann, wobei die Rohrleitung (62) wenigstens abschnittsweise spiralförmig ausgebildet ist.

2. Verdampfer (1) nach Anspruch 1,
**dadurch gekennzeichnet, dass**
die Einspritzeinrichtung (12) eine Düse (18, 19) aufweist, welche die Substanz auf eine Außenoberfläche der Leitung aufbringt.

3. Verdampfer (1) nach wenigstens einem der vorangegangenen Ansprüche,
**dadurch gekennzeichnet, dass**
die Leitung (62) wenigstens abschnittsweise eine kegelförmige Oberfläche (62a) ausbildet.

4. Verdampfer (1) nach wenigstens einem der vorangegangenen Ansprüche,
**dadurch gekennzeichnet, dass**
wenigstens ein weiterer Bestandteil des Gehäuses (2) durch ein fließfähiges Medium erwärrnbar ist.

5. Verdampfer (1) nach wenigstens einem der vorangegangenen Ansprüche,
**dadurch gekennzeichnet, dass**
die Vorrichtung (1) unterhalb der Leitung (62) einen beheizbaren Abschnitt (80) aufweist.

6. Verdampfer (1) nach wenigstens einem der vorangegangenen Ansprüche,
**dadurch gekennzeichnet, dass**
die Einspritzeinrichtung (12) als Ringdüse (19) ausgebildet ist.

7. Verdampfer (1) nach wenigstens einem der vorangegangenen Ansprüche,
**dadurch gekennzeichnet, dass**
unterhalb der Leitung (62) ein doppelwandiger Boden (90) vorgesehen ist, wobei zwischen den Wänden (92, 94) dieses Bodens ein fließfähiges Medium führbar ist

8. Verdampfer (1) nach Anspruch 7,
**dadurch gekennzeichnet, dass**
sich der doppelwandige Boden (90) in einer Strömungsrichtung des fließfähigen Mediums an die Leitung (62) anschließt.

9. Verdampfer (1) nach wenigstens einem der vorangegangenen Ansprüche,
**dadurch gekennzeichnet, dass**
die Heizeinrichtung (8) eine Öffnung (63) ausbildet, welche unterhalb der Einspritzeinrichtung (12) angeordnet ist

10. Verfahren zum Sterilisieren von Behältnissen, wobei in einen Verdampfer (1) ein gasförmiges Medium eingeführt wird und mittels einer Einspritzeinrichtung (12) eine Substanz eingespritzt wird, welche Wasserstoffperoxid (H₂O₂) enthält und wobei diese Substanz an einer Heizeinrichtung (8) verdampft wird und über eine zweite in dem Gehäuse (2) angeordnete Öffnung, (4) das entstehende Gemisch aus dem gasförmigen Medium und der verdampften Substanz abgeführt wird,
**dadurch gekennzeichnet, dass**
die Heizeinrichtung (8) mittels eines fließfähigen Mediums erwärmt wird wobei die Heizeinrichtung (8) eine von dem fließfähigen Medium durchströmte Leitung (62) aufweist, die von der Substanz unmittelbar beaufschlagt wird, wobei die Rohrleitung (62) wenigstens abschnittsweise spiralförmig ausgebildet ist.

## Claims

1. A vaporizer (1) for the sterilization of containers with a housing (2), with a first opening (11) provided in the housing (2), in order to supply a gaseous medium to the housing (2), with an injection device (12), in order to inject a substance which contains hydrogen peroxide () into the housing (2), with a heating device (8) arranged inside the housing (2) in order to vaporize the hydrogen peroxide and with a second opening (4) provided in the housing (2) in order to remove a mixture of the gaseous medium and the vaporized hydrogen peroxide out of the housing (2), **characterized in that**
the heating device (8) has a line (62) through which a flowable medium can flow, wherein this line (62) is used for heating the heating device (8) and is capable of being acted upon by the substance in such a way that the hydrogen peroxide (H₂O₂) containing substance can reach directly to an outer surface of the line wherein the pipeline (62) is shaped at least in sections spirally.

2. A vaporizer (1) according to claim 1,
**characterized in that**
the injection device (12) has a nozzle (18, 19) which applies the flowable medium to an outer surface of the line.

3. A vaporizer (1) according to at least one of the preceding claims, **characterized in that**
the line (62) forms a tapered surface (62a) at least locally.

4. A vaporizer (1) according to at least one of the preceding claims, **characterized in that**
at least one further component part of the housing (2) is capable of being heated by a flowable medium.

5. A vaporizer (1) according to at least one of the preceding claims, **characterized in that**
below the line (62) the apparatus (1) has a portion (80) capable of being heated.

6. A vaporizer (1) according to at least one of the preceding claims, **characterized in that**
the injection device (12) is designed in the form of an annular nozzle (19).

7. A vaporizer (1) according to at least one of the preceding claims, **characterized in that**
a double-walled bottom (90) is provided below the line (62), wherein a flowable medium is capable of being conveyed between the walls (92, 94) of this bottom.

8. A vaporizer (1) according to claim 7,
**characterized in that**
the double-walled bottom (90) is adjacent to the line (62) in a flow direction of the flowable medium.

9. A vaporizer (1) according to at least one of the preceding claims, **characterized in that**
the heating device (8) has an opening (63) which is arranged below the injection device (12).

10. A method of sterilizing containers, wherein a gaseous medium is introduced into a vaporizer (1) and a substance which contains hydrogen peroxide (H₂O₂) is injected by means of an injection device (12), wherein this substance is vaporized on a heating device (8) and the resulting mixture of the gaseous medium and the vaporized substance is removed by way of a second opening (4) arranged in the housing (2), **characterized in that**
the heating device (8) is heated by means of a flowable medium wherein the heating device (8) has a line (62) through which the flowable medium flows and which is directly acted upon by the substance wherein the pipeline (62) is shaped at least in sections spirally.

## Revendications

1. Évaporateur (1) destiné à stériliser des récipients, comprenant un boîtier (2), une première ouverture (11) ménagée dans le boîtier (2), afin d'apporter un milieu gazeux au boîtier (2), un dispositif d'injection (12), afin d'injecter une substance dans le boîtier (2), laquelle contenant du peroxyde d'hydrogène (H₂O₂), un dispositif de chauffage (8) agencé à l'intérieur du boîtier (2) et servant à faire évaporer le peroxyde d'hydrogène et une deuxième ouverture (4) ménagée dans le boîtier (2), afin d'évacuer du boîtier (2) un mélange composé du milieu gazeux et du peroxyde d'hydrogène évaporé,
**caractérisé en ce que**
le dispositif de chauffage (8) comprend une conduite (62) pouvant être traversée par un milieu coulant, dans lequel cette conduite (62) sert à chauffer le dispositif de chauffage (8) et peut directement être soumise à l'action de la substance, de telle sorte que la substance contenant du peroxyde d'hydrogène (H₂O₂) peut parvenir directement sur une surface extérieure de la conduite, dans lequel la conduite (62) est au moins en partie en forme de spirale.

2. Évaporateur (1) selon la revendication 1,
**caractérisé en ce que**
le dispositif d'injection (12) comprend une buse (18, 19), laquelle applique la substance sur une surface extérieure de la conduite.

3. Évaporateur (1) selon au moins l'une des revendications précédentes,
**caractérisé en ce que**
la conduite (62) comprend au moins en partie une surface conique (62a).

4. Évaporateur (1) selon au moins l'une des revendications précédentes,
**caractérisé en ce que**
au moins un autre composant du boîtier (2) peut être chauffé par un milieu coulant.

5. Évaporateur (1) selon au moins l'une des revendications précédentes,
**caractérisé en ce que**
le dispositif (1) comprend en dessous de la conduite (62) une partie chauffante (80).

6. Évaporateur (1) selon au moins l'une des revendications précédentes,
**caractérisé en ce que**
le dispositif de chauffage (12) est réalisé sous la forme d'une buse annulaire (19).

7. Évaporateur (1) selon au moins l'une des revendications précédentes,
**caractérisé en ce que**
un fond à double paroi (90) est prévu en dessous de la conduite (62), dans lequel un milieu coulant peut être guidé entre les parois (92, 94) de ce fond.

8. Évaporateur (1) selon la revendication 7,
**caractérisé en ce que**
le fond à double paroi (90) est dans le prolongement de la conduite (62) dans une direction d'écoulement du milieu coulant.

9. Évaporateur (1) selon au moins l'une des revendications précédentes,
**caractérisé en ce que**
le dispositif de chauffage (8) constitue une ouverture (63), laquelle est ménagée en dessous du dispositif d'injection (12).

10. Procédé de stérilisation de récipients, dans lequel un milieu gazeux est introduit dans un évaporateur (1) et une substance, laquelle contient du peroxyde d'hydrogène (H₂O₂), étant injectée au moyen d'un dispositif d'injection (12) et dans lequel cette substance étant évaporée sur un dispositif de chauffage (8) et le mélange ainsi produit composé du milieu gazeux et de la substance évaporée étant évacué par une deuxième ouverture (4) ménagée dans le boîtier (2),
**caractérisé en ce que**
le dispositif de chauffage (8) est chauffé au moyen d'un milieu coulant, dans lequel le dispositif de chauffage (8) comprend une conduite (62) traversée par le milieu coulant, qui est directement soumise à l'action de la substance, dans lequel la conduite (62) est au moins en partie en forme de spirale.
